# EUROPEAN PATENT SPECIFICATION

(11) **EP 3 514 546 B1**
(45) Date of publication and mention of the grant of the patent: **01.03.2023**
(21) Application number: 19153335.5
(22) Date of filing: 23.01.2019
(51) Int. Cl.: G01N 33/68

(54) **METHOD AND KIT FOR CARRYING OUT THE EARLY DIAGNOSIS OF PARKINSON'S DISEASE IN VITRO**
VERFAHREN UND VORRICHTUNG ZUR DURCHFÜHRUNG DER FRÜHDIAGNOSE DER PARKINSON-KRANKHEIT IN VITRO
PROCEDE ET KIT PERMETTANT DE FAIRE LE DIAGNOSTIC TOTAL DE LA MALADIE DE PARKINSON IN VITRO

(30) Priority: 23.01.2018 IT 201800001696
(43) Date of publication of application: 24.07.2019
(73) Proprietor: UNIVERSITA' DEGLI STUDI DI CAGLIARI, 09124 Cagliari (IT)
(72) Inventor: COCCO, CRISTINA, 09042 MONSERRATO (IT)
(74) Representative: Primiceri, Maria Vittoria

(56) References cited:
- WO-A1-2011/139773
- CARLA BRANCIA ET AL: "VGF Protein and Its C-Terminal Derived Peptides in Amyotrophic Lateral Sclerosis: Human and Animal Model Studies", PLOS ONE, vol. 11, no. 10, 13 October 2016 (2016-10-13), page e0164689, XP055485104, DOI: 10.1371/journal.pone.0164689
- CRISTINA COCCO ET AL: "Distribution of VGF peptides in the human cortex and their selective changes in Parkinson's and Alzheimer's diseases : VGF peptides in the human cortex", JOURNAL OF ANATOMY., vol. 217, no. 6, 12 October 2010 (2010-10-12), pages 683-693, XP055485113, GB ISSN: 0021-8782, DOI: 10.1111/j.1469-7580.2010.01309.x
- BARBARA NOLI ET AL: "VGF Changes during the Estrous Cycle: A Novel Endocrine Role for TLQP Peptides?", PLOS ONE, vol. 9, no. 10, 3 October 2014 (2014-10-03), page e108456, XP055485143, DOI: 10.1371/journal.pone.0108456
- CRISTINA COCCO ET AL: "VGF Involvement on Parkinson's disease", ITALIAN JOURNAL OF ATANOMY AND EMBRYOLOGY, vol. 119, no. 1suppl, 1 December 2014 (2014-12-01), page 47, XP055485188, IT ISSN: 1122-6714, DOI: doi:10.13128/IJAE-15878
- FILOMENA DAMATO ET AL: "VGF peptides upon osmotic stimuli: Changes in neuroendocrine regulatory peptides 1 and 2 in the hypothalamicpituitary-axis and plasma", JOURNAL OF CHEMICAL NEUROANATOMY, CHICHESTER, GB, vol. 44, no. 2, 3 May 2012 (2012-05-03), pages 57-65, XP028428401, ISSN: 0891-0618, DOI: 10.1016/J.JCHEMNEU.2012.05.001 [retrieved on 2012-05-18]
- GIAN-LUCA FERRI ET AL: "VGF: An inducible gene product, precursor of a diverse array of neuro-endocrine peptides and tissue-specific disease biomarkers", JOURNAL OF CHEMICAL NEUROANATOMY, CHICHESTER, GB, vol. 42, no. 4, 12 May 2011 (2011-05-12), pages 249-261, XP028104744, ISSN: 0891-0618, DOI: 10.1016/J.JCHEMNEU.2011.05.007 [retrieved on 2011-05-20]
- ALICE S. CHEN-PLOTKIN ET AL: "Plasma epidermal growth factor levels predict cognitive decline in Parkinson disease", ANNALS OF NEUROLOGY., vol. 69, no. 4, 29 November 2010 (2010-11-29), pages 655-663, XP055485177, BOSTON, US ISSN: 0364-5134, DOI: 10.1002/ana.22271
- MIN SHI ET AL: "Plasma exosomal [alpha]-synuclein is likely CNS-derived and increased in Parkinson's disease", ACTA NEUROPATHOLOGICA., vol. 128, no. 5, 6 July 2014 (2014-07-06), pages 639-650, XP055225837, BERLIN, DE ISSN: 0001-6322, DOI: 10.1007/s00401-014-1314-y
- EL-AGNAF OMAR M A ET AL: "Detection of oligomeric forms of alpha-synuclein protein in human plasma as a potential biomarker for Parkinson's disease", FASEB JOU,, vol. 20, no. 3, 1 March 2006 (2006-03-01), pages 419-425, XP009109458, DOI: 10.1096/FJ.03-1449COM

## Description

### Technical field

The present invention relates to a method for performing in a simple, safe and economical way the early *in vitro* diagnosis of Parkinson's disease.

Also described herein but not part of the invention is a diagnostic kit for performing said diagnosis early, simply and economically.

### State of the Art

### General description

Currently, in Italy there are 300,000 persons affected by Parkinson's Disease (or PD), mainly males (1.5 times more than females), with an average age of onset between 59 and 62 years. This disease occurs with tremors, rigidity, slow movements, reaching even the complete paralysis in its advanced phase. These symptoms are due to the death of the neurons that control voluntary movements and that produce a neurotransmitter called dopamine; these symptoms are attenuated with a levodopabased therapy (levodopa or L-DOPA is an intermediate amino acid in the biosynthetic pathway of dopamine, into which it is converted by brain cells). Parkinson's disease is diagnosed both by an accurate anamnesis based on the patient's clinical history and with the aid of neurological tests to be combined with nuclear medicine tests. As far as known to the present inventors, unfortunately, there is no simple test that can clearly identify the disease and discriminate it from the other ones in its early stages. Actually, the probability of obtaining a reliable diagnosis at an early stage is very low because, as a rule, at the start the symptoms are not clear-cut, but they become so as the disease progresses, manifesting themselves also in a more differentiated way.

Among the methodologies currently used to diagnose PD, the following techniques can be mentioned:
- brain magnetic resonance imaging;
- positron emission tomography (or PET), which works with ¹⁸F marked fluorodeoxyglucose as a slightly radioactive tracer;
- MIBG scintigraphy (which uses metaiodobenzylguanidine, or *iobenguane,* as a radio-marker), which shows the innervation of the heart with autonomous nervous fibres.

There exsist also pharmacological techniques, for example:
- verifying the response to levodopa (test with L-Dopa) or a single administration of apomorphine (by subcutaneous injection).

### Deficiencies of the State of the Art

The above described tests support the neurological differential diagnosis, however they are not sufficiently precise (often they do not selectively discriminate Parkinson's disease from the other neurodegenerative diseases). Moreover, they are ill-suited for early diagnosis, because as a rule they identify a pathological result only at a more advanced stage of the disease. In particular, especially when the clinical outcomes are uncertain or blurred, obtaining a reliable diagnosis may require 6 months or even one year, with the concurrent further increase of the neurodegeneration processes involving dopaminergic neurons. Indeed, at the time of diagnosis, often 80% or even more of dopaminergic neurons are already irremediably compromised.
a). In Brancia, C. et al., "VGF Protein and its C-terminal derived peptides in Amyotrophic Lateral Sclerosis: Human and Animal Model Studies", PLOS ONE, vol. 11, no. 10, 13 October 2016, page e0164689, reference is made to the same measurement of specific peptides (of the C-terminal portion of VGF protein) but patients are not affected by Parkinson's disease, it is the study of a different pathology.
b). In Cocco, C. et al., "Distribution of VGF peptides in the human cortex and their selective changes in Parkinson's and Alzheimer's diseases: VGF peptides in the human cortex", Journal of Anatomy, vol.217, no.6, 12 October 2010, pages 683-693, reference is made to the same measurement of specific peptides (of the C-terminal portion of VGF protein) but using human brains (*post mortem* from Parkinson's and Alzheimer's patients). Human brains are a different tissue from plasma and hence they entail different tests and experiments (for example: a different processing from that of plasma, the use of specific areas because brain cannot be used *in toto,* etc.). Moreover, the use of human brains in no case can be useful at diagnostic level.
c). In Noli, B. et al., "VGF changes during the Estrous Cycle: a novel endocrine role for TLQP peptides?", PLOS ONE, vol. 9, no. 10, 3 October 2014, page e108456, reference is made to the same measurement of specific peptides (of the C-terminal portion of VGF protein) using both plasma and other tissues, but in this case all tissues are of animal origin. This involves the use of primary antibodies against the rat sequence which is different from the human VGF sequence used for the present invention.
d). In Cocco, C. et al., "VGF involvement on Parkinson's disease", Italian Journal of Anatomy and Embryology, vol.119, no. 1suppl, 1 December 2014, page 47, reference is made to the measurement of peptides of VGF protein with the use of plasma originating from patients affected by confirmed Parkinson's disease. In spite of this, reference is made to an antibody against the C-terminal portion of VGF not to an antibody directed against the C-terminal nonapeptide of VGF protein. The specificity of the invention and its relevance in the diagnosis of Parkinson's disease is indeed closely tied to the type of antibody used specifically produced against the C-terminal nonapeptide of VGF protein. Indeed antibodies and peptides different from the nonapeptide of the present invention (even by a single amino acid) could lead to different or even opposite results. In addition, reference is made to a very small and imprecise population of sick patients and the decrease is not referred to an initial stage of the disease (which is a characterising item of the present invention, because it is fundamental for the diagnostic aspect).
e). In Damato, F. et al., "VGF peptides upon osmotic stimuli: changes in neuroendocrine regulatory peptides 1 and 2 in the hypothalamicpituitary-axis and plasma", Journal of Chemical Neuroanatomy, Chichester, GB, vol. 44, no. 2, 3 May 2012, pages 57-65, reference is made to the same measurement of specific peptides (of the C-terminal portion of VGF protein) using human plasma, but in this case plasma comes only from patients affected by diabetes, a pathology that is not included among the neurodegenerative ones and does not involve the central nervous system, hence is complete different from Parkinson's.
f). In Ferri, G.L. et al., "VGF: an inducible gene product, precursor of a diverse array of neuro-endocrine peptides and tissue-specific disease biomarkers", Journal of Chemical Neuroanatomy, Chichester, GB, vol. 42, no. 4, 12 May 2011, pages 249-261, all published results pertaining to VGF protein and its peptides are summarised, but there are no indications about Parkinson's disease except for the Cocco, C. et al. paper, discussed above.
g). In WO2011/139773, reference is made to 11 proteins identified as possible biomarkers for Parkinson's disease, but none of them corresponds to VGF protein (nor VEGF, different both as sequence and as function). Moreover, these proteins are associated with the Parkinson's patients' risk of developing dementia (which can emerge in a variable manner during the course of the disease). No reference is specifically made to diagnosis.

### Importance of Early Diagnosis of PD

The early diagnosis of Parkinson's disease, therefore, is still a crucial challenge for neurologists because, if a simple and safe methodology for its acquisition existed, it would allow to recognise the disease when the clinical symptoms are still uncertain or blurred and, most of all, when degeneration is at an incipient and/or minimal stage. It is evident that the quality of life of PD patients would significantly improve if they could start the therapy (for example with levodopa) already at the early stages of the disease, or at the first suspicion. In this way, the drug would be able to protect healthy neurons, slow neurodegeneration and also controll the symptoms avoiding the undesired long-term effects of the pharmacological therapy, with the benefit of at least in part preventing, or at least delaying, the onset and/or the development of the pathology itself.y

### Technical Problem

Hence, there remains a great request by the physicians for a method that is reliable, easily implemented, safe for the patient (and, possibly, also less costly than the currently used methods), to diagnose PD at an early stage, thus allowing advantageously to intervene early with an adequate therapy that possibly blocks and/or significantly slows down the progress of the disease and/or of its symptoms, while improving the quality of life of the patient.

A purpose of the present invention is to provide an adequate response to the technical problem described above.

### Brief Description of the Invention

### Preamble

With the intent of finding new possible bio-markers for Parkinson's disease, the changes that occur in the levels of various proteins and/or neurotransmitters, in particular, in cerebrospinal fluid or CSF, were studied (Jimenez *et al.,* 2014, (**1**)), but with few feedbacks in blood (Lin *et al.,* 2014, (**2**); Scalzo P. *et al.,* 2010, (**3**)). In this regard, concerning, for example, VGF protein (a neuropeptide, that acts as a local chemical mediator, inducible by the nerve growth factor), there are known publications of various types, based on multiple studies, that show how different fragments of VGF protein (which contain sequences at various levels of the precursor protein, especially astride the N-terminal portion) undergo changes in various neurodegenerative diseases, for example Alzheimer's disease (Carrette O., *et al.,* 2003, (**4**) and dementia (Ruetschi U., *et al.,* 2005, (**5**)). Moreover, there is International Patent Application WO 2016/044665 A1 (in the name of Patrizia Fanara, of KINEMED, INC., California, U.S.A. (**6**)) directed at a method for diagnosing Parkinson's disease, which uses VGF protein as a kinetic biomarker. In this patent, the invention is based on the initial administration to the patient of a substrate marked with an isotope (an aqueous drink called "heavy water" containing the isotope D₂O or ²H₂O) for a certain period of time (approximately 14-21 days). The measurement is carried out on samples of the cerebrospinal fluid drawn from the patient, after administration of aforesaid drink, to quantify the presence of the VGF protein bonded to the isotope. The samples of the cerebrospinal fluid are examined using particular techniques, such as: "liquid scintillation counting for ³H"; "mass spectrometry"; "laser spectroscopy"; "NMR spectroscopy" (for the measurements see also: Jungas R.L., 1968, (7) Biochemistry. 1968 7:3708-17). In general, it has been found that a significantly lower level of the VGF protein with respect to the level measured in healthy patients is an index of the start and/or of the development of the disease. The teaching of aforesaid patent application is directed, substantially, at using the cerebrospinal fluid drawn from the patient to carry out the *in vitro* measurement of the peptides derived from VGF and/or from VGF protein.

However, it must be underlined that the administration to the patient of the substrate marked with an isotope, i.e. the aforesaid "heavy water" aqueous drink, is a long procedure (it requires approximately 14-21 days of administration) and that, therefore, it is not free of risks for the patient (see, for example, the entry *"Heavy water"* in Wikipedia under *Paragraph* 5. *Toxicity*)*.* Moreover, the administration is followed by drawing the cerebrospinal fluid, which is also a highly invasive procedure and dangerous for the patient. In addition, the technologies for measuring the VGF protein are not simple to use. Consequently, it is not desirable for the routine, easy, safe, reproducible and relatively economical performance of a test for the early identification of the onset and/or of the presence of Parkinson's disease.

### Summary of the Invention

The Applicant now has unexpectedly found that the determination of the levels of the portion containing the C-terminal peptide of VGF protein present in blood plasma, by carrying out a simple Enzyme-Linked ImmunoSorbent Assay (ELISA), specific for said C-terminal portion, followed by an equally simple spectrophotometric final determination, is able to provide an adequate response to the technical problem highlighted above.

Therefore, it is an object of the present invention a method for determining *in vitro* the levels of said portion containing the C-terminal peptide of VGF protein present in the plasma of an individual, as set forth in the appended independent claim.

Also described herein but not part of the invention is a diagnostic kit for determining *in vitro* the levels of said portion containing the C-terminal peptide of VGF protein present in the plasma of an individual.

The invention is defined in the appended claims.

Preferred embodiments of the present invention are set forth in the appended dependent claims.

The preferred embodiments of the present invention as set forth in the following description are provided therein solely by way of absolutely non-limiting example, of the field of application of the present invention, which will be immediately clear to the person skilled in the art.

### Detailed Description of the Invention

### Definition

For simplicity, the term *"levels of VGFprotein",* as used in the present description and in the appended claims, means both VGF protein as such (i.e., whole) and the peptide fragments deriving therefrom and containing the C-terminal portion thereof, and a mix thereof, possibly present in the plasma of an individual or of an animal.

### Description

It is an aspect of the present invention a method for determining *in vitro* the levels of VGF protein, as defined above, present in the plasma of a human subject, said method consisting of the following steps:
1) subjecting the plasma of said subject to an ELISA assay that uses antibodies that specifically mark the VGF protein C-terminal peptide, said C-terminal nonapeptide being represented by the amino acid sequence **-I EHVL LRRP** (in which the letters correspond to the acronyms of the individual component amino acids);
2) calculating the VGF protein levels by the optical density spectrophotometric determination of the samples obtained at the end of the ELISA immuno-enzymatic reaction of step 1), as described below.

In said step 1), while performing the ELISA assay, the plasma of the patient is made to react, preferably in a "multiwell" plate (i.e. preferably with 96 wells; for example, obtained from Nunc, Milan, Italy), with an appropriate primary antibody (Ab1), that specifically marks the C-terminal nanopeptide of human VGF **(-I EHVL LRRP),** in the specific conditions described in the following *Experimental Section,* to give the corresponding protein-primary antibody conjugate. Subsequently, the protein-primary antibody conjugate thus obtained is made to react with an appropriate biotinylated secondary antibody (Ab2) that marks said primary antibody, and with streptavidin peroxidase, in the specific conditions described in the following *Experimental Section,* to give the corresponding desired conjugate protein-primary antibody-biotinylated secondary antibody and streptavidin peroxidase.

At the end of said immuno-enzymatic reaction, the spectrophotometric determination of the optical density of the resulting samples is carried out, in the specific conditions described in the following *Experimental Section,* to give the desired concentration of the levels of said VGF protein present in the plasma of the patient.

### Experimental Section

### ELISA

The competitive ELISA assay was carried out in the following way.

At least one multiwell plate, for example with 96 wells (Nunc, Milan, Italy), was coated with the synthetic nonapeptide -**I EHVL LRRP** (commercially available, for example, from Jackson, West Grove, PA, U.S.A.) and treated with a phosphatebuffered saline solution (PBS, containing 9% donkey serum, 20 nM aprotinin, 1mg/mL EDTA), for 2 hours at room temperature. Primary incubations, with the primary antibody (Ab1) that specifically marks the portion containing the C-terminal peptide of human VGF (commercially available, for example, from Jackson, West Grove, PA, U.S.A.), were carried out at ambient temperature in duplicate, including standard serial dilutions of the same synthetic peptide **-I EHVL LRRP** used to treat the plates and commercially available, in parallel with those of the plasma samples originating from the PD patients and from the controls. At the end, the biotinylated secondary antibodies (Ab2), which mark the corresponding primary antibody previously used (commercially available, for example, from Jackson, West Grove, PA, U.S.A.), the streptavidin-peroxidase conjugate (Biospa, Milan, Italy), and tetramethylbenzidine (TMB X-traKem-EN-Tec, by Taastrup, Denmark) were used at room temperature by adding them to the protein-primary antibody conjugate. Subsequently, after approximately 10 minutes, the reaction was arrested with HCl (1 mol/L) and the resulting optical density of the samples was measured at 450 nm using a multilabel plates reader spectrophotometer (for example by *Chameleon: Hidex, Turku,* Finland). The recovery of the synthetic peptide(s) added to plasma at the beginning was found to be 85%.

### Statistical Analysis

For the ELISA data, statistical analyses were carried out by a one-way ANalysis Of VAriance (ANOVA), followed by multiple post hoc comparison tests (Student-Newman-Keul test), or by two-tailed Student's t-test, as appropriate, using the StatistiXL software (Windows version of *Microsoft's sophisticated Excel spreadsheet*)*.* For all analyses, a value of P < 0.05 was considered significant.

### Results

The present inventors have analysed the levels of VGF protein both in control subjects, healthy (n=21), and in subjects affected by PD (total: n=64) not yet pharmacologically treated (n=23) or subjected to pharmacological medication (prevalently, L-Dopa) for a relatively short period of time (1-6 years; n=23) and for a long period of time (7-25 years; n=18).

The VGF protein levels found in the healthy control subjects vary between 1,400 and 1,600 pmol/mL.

With respect to the healthy patients, in PD patients without treatment, the VGF protein levels decreased markedly, with a decrease > 50%, p ≤ 0.0001 with respect to the controls. They continue to be significantly reduced also under the short time treatment (1-6 years) but the reduction is more evident in the untreated ones. Instead, they increased, returning to similar values as those of the healthy controls, after a prolonged treatment with L-Dopa (p ≤ 0.05). Summarising, the present inventors have thus unexpectedly shown that the levels of VGF protein in plasma decrease to a very significant extent in the subjects in which PD is at an initial development stage (decrease > 50% with respect to healthy control subjects, as indicated above). Moreover, they have demonstrated that a prolonged treatment with L-Dopa is able to restore normal levels of VGF. Instead, in other studied patients, affected by other neurodegenerative diseases, the VGF levels do not decrease at an early stage. Another interesting aspect is that the findings in humans were also confirmed in rats by using the same conditions but with the peptides of the C-terminal sequence of rat (the rat C-terminal nanopeptide is: **-I EHVL LHRP**) and with the related specific antibodies. In rats the decrease of VGF levels has even been seen in that specific area of the brain, where the dopaminergic neurons die (the *substantia nigra*)*.*

As a consequence of all that has been illustrated above, an early diagnosis of Parkinson's disease *in vitro,* in human and/or animal plasma, was found possible and shown to be extremely promising and useful thanks to the use of the method of the present invention, as described above, and of the related application kit, described hereinafter.

Also described herein but not part of the invention is a diagnostic kit for carrying out the measurement *in vitro,* in the plasma of an individual and/or of an animal, of the levels of the portion containing the C-terminal nonapeptide of VGF protein, according to the method of the invention as described and claim herein, which identifies the early stages of Parkinson's disease.

Said kit comprises, or consists of
1) at least one multiwell plate (for example, with 96 wells) pre-treated with the **-I EHVL LRRP** synthetic nonapeptide in the case of human plasma (or **-I EHVL LHRP** in the case of samples of rat or mouse plasma, for which the sequence is the same);
2) a first test tube, or a first equally suitable container, with a removable seal, containing the primary antibody (Ab1) of commercial origin mentioned above that specifically marks the VGF protein C-terminal peptide, in a lyophilized form (because it is preserved better and for a longer time); moreover, on, or coupled to, said first test tube will be shown the required volume of 0.1% PBS-BSA with which to reconstitute it to obtain a starting dilution, i.e. a stock, of 1:10;
3) a second test tube, or a second equally suitable container, with a removable seal, containing the biotinylated secondary antibody (Ab2) of commercial origin mentioned above that specifically marks the peptide-primary antibody conjugate, in a lyophilized form (because it is preserved better and for a longer time); moreover, on, or coupled to, said second test tube will be shown the required volume of 0.1% PBS-BSA with which to reconstitute it to obtain a starting dilution, i.e. a stock, of 1:10;
4) a third test tube, or a third equally suitable container, with a removable seal, containing the streptavidin of commercial origin that specifically marks the peptide-primary antibody-biotinylated secondary antibody conjugate, in a lyophilized form (because it is preserved better and for a longer time); moreover, on, or coupled to, said third test tube will be shwn the required volume of 0.1% PBS-BSA with which to reconstitute it to obtain a starting dilution, i.e. a stock, of 1:10;
5) a fourth test tube, or a fourth equally suitable container, with a removable seal, containing the synthetic peptide (**-I EHVL LRRP** for human plasma samples, or **-I EHVL LHRP** for rat and mouse plasma/tissues) for realizing the standard curve; moreover, on, or coupled to, said fourth test tube will be shown a concentration in nano moles/mL (reached by measuring the peptide dissolved in bi-distilled water with spectrophotometer at 214 nanometers) and, hence, the volume of bi-distilled water that has to be added to obtain a starting dilution, i.e. a stock, of 1:10.

While, in a particularly preferred embodiment of the invention, in the kit described herein the multiwell plate is a plate that has already been pre-treated with the synthetic peptide as per at point 1) above (and, hence, anyone who purchases the kit has no need to carry out any pre-treatment), said pre-treatment of said plate consists, in general, of:
- incubating the plate with the desired peptide overnight at 4°C under constant agitation, at a concentration of 5 pmol/mL, diluted with 9% donkey serum; then
- washing the plate with PBS-tween (a commercial solution of PBS plus tween, which is a surfactant that serves to favour the antigen-antibody bond, from Merck KGaA, Darmstadt, Germany), and incubating it with 9% donkey serum for 2 h at room temperature under agitation.

The pre-treated plates are stored up at 4°C.

With regard to human or animal plasma, the experimental work of the present invention was carried out using samples of plasma preserved at low temperature, with the commonly known preservation techniques. In any case, the plasma samples can be prepared and/or preserved through procedures that are well known to all persons skilled in the art and, consequently, they will not be described herein because they are not a part of the invention.

### Performing the Assay According to the Invention

a) Before being used, the pre-treated plates are washed with PBS-tween at least three times.
b) The curve of the peptide is then prepared; scalar concentrations are prepared of the C-terminal synthetic peptide of VGF **(-I EHVL LRRP** for human serum samples, in the fourth test tube of the kit) which is diluted in 9% donkey serum so as to obtain a final concentration in the plate that ranges from 0.005 to 500 pmol/mL. Since 1/5 of the total volume is placed in each well for each of these solutions, the starting (stock) solution will have to be 5 times more concentrated.
c) The dilutions of plasma samples are then prepared. 2 different final dilutions are to be obtained, i.e. 1:40 and 1:80. Since the volume of plasma that is inserted in each well is 1/5 of the total volume, the real concentration of plasma will have to be 5 times more concentrated.
d) The dilution of the primary antibody (Ab1) is then prepared; the primary antibody Ab1 (anti VGF C-terminal, defined above) has to have a final dilution of 1:16000. Since the volume inserted in each well with respect to the total volume is a half, the solution of the antibody diluted in 9% donkey serum will have to be twice as concentrated as the final dilution in the plate (1:8000).
e) After preparing all the solutions, 30 µL of 9% donkey serum are placed in all wells, then 20 µL of human (or rat, as needed) antigen (the aforesaid synthetic peptide) are added in 6 wells, to build up the standard curve made with dilutions ranging from 0.005 to 500 pmol/mL, in duplicate (hence, using 12 wells in all), while in the other wells (the number of wells depends on the number of plasma samples to be measured, but it is assumed that the entire plate will be used) are inserted the plasma samples (20 µL per well, in duplicate, at 1:40, 1:80 dilutions). At the end, in the wells thus prepared are added 50 µL of primary antibody produced in rabbit, dilution 1:16000 (total volume of solution for each well = 100 µL). The plate thus prepared is kept for 3h under constant agitation at room temperature. It should be kept in mind that in some wells (at least 2, but also 4, as desired) the primary antibody and the sample (or standard) are omitted and in others only the sample (or standard) is omitted, incubating the serum alone instead of both of them (to have the negative and positive controls of the secondary antibody).
f) The solution of the secondary antibody Ab2 (anti-rabbit, biotylinated) in 9% donkey serum (at the dilution of 1:10,000) is then prepared.
g) Once the 3 hours of point e) above elapsed, the plate is washed with PBS-tween at least three times; then the antibody Ab2, as freshly prepared at point f) above, is inserted into the wells (100 µL per well) and left to incubate for 1 hour at room temperature; then, once the hour of incubation elapsed, the plate is rinsed 3 times with PBS-tween.
h) The solution of streptavidin-peroxidase (HRP-conj) in 0.01% PBS-BSA is then prepared at a dilution of 1:10,000; it is then inserted into the plate (100 µL/well) and 30 minutes are allowed to elapse, maintaining the plate under constant agitation at room temperature.
i) Once the 30 minutes of point h) above elapsed, the plate is rinsed 3 times with PBS-tween; then, 100 µL/well of TMB (tetramethylbenzidine) are inserted into and allowed to act for 10 minutes under agitation at room temperature.
j) After the 10 minutes of point i) above elapsed, the reaction is stopped with 100 mL/well of HCl 1M (without washings); the plate is then read in absorbance (at 495 nm) by a spectrophotometer suited for reading plates, as described above in the description.

The measurement with the plates treated as described above has a total duration of approximately 8 hours and, as already described, it takes place with a spectrophotometer for reading multiwell plates (like, for example, the one described in the preceding *Experimental Section*)*.*

Both the reagents necessary to carry out the aforesaid spectrophotometric reading (i.e. tetramethylbenzidine and hydrochloric acid) and the spectrophotometer for reading the multiwell plates are not, obviously, part of the kit described herein, but in general they are commonly present in the biomedical laboratories.

Concerning the characteristics of the method, as well as of the kit to which said method applies, the following characterising properties were observed:
- a 7-15% variation among the different assays (inter-assay; number of experiments 6);
- a 5-8% variation within an individual assay (intra-assay; number of experiments 11).

The kit was validated *in vitro* in the human species, in mice, in rats, and also in the bovine species (data not reported), using plasma samples.

As demonstrated above, the kit described herein has been demonstrated to be particularly advantageous to carry out, applying the method of the present invention, the *in vitro* diagnosis, at an early stage, of Parkinson's disease, allowing to obtain the desired results in a safe, repeatable, and economical way starting from the plasma of an individual and/or of an animal. Thus, it has also allowed to start therapy at an early stage of the disease, potentially obtaining the advantages described above, significantly improving the quality of life of the patient.

Summarising, to better clarify what has been expressed above, the kit described herein provides for the use of plates pre-treated with the specific antigen (the aforesaid C-terminal portion of VGF consisting of the above nine amino acids). Into the plates are then added, in the order: (i) scalar concentrations of the C-terminal protein of nine amino acids (obtaining the standard curve); (ii) the plasma samples to be tested at different concentrations; (iii) the "blank" controls, i.e. solutions without proteins or sample; (iv) primary antibodies (for example, produced in the laboratory of the inventor) against the nonapeptide of the C-terminal portion of the VGF protein; (v) commercially available secondary antibodies; (vi) streptavidin conjugated with peroxidase, commercially available. Last, the measurement of the quantities of the C-terminal peptides of VGF (in picomoles/mL) thanks to the reaction of the peroxidase is carried out by means of a spectrophotometer at 450 nm (an instrument that is commonly used by many diagnostic laboratories).

In light of the above description, a characteristic aspect of the present invention is the use of a consolidated technique, such as ELISA, to measure specific peptides (those of the C-terminal portion of the VGF protein) detected exclusively by means of a specific antibody produced against the nine amino acids of the C-terminal portion of the VGF protein that are useful for the purposes of the early diagnosis of Parkinson's disease. Therefore, any possible use of the kit for other purposes (for example to diagnose other diseases), or with other tissues other than plasma, or with the plasma itself but originating from different species, differs markedly from the invention, because the unexpected innovation of the present invention is derived from specific scientific researches and directed at the early diagnosis of Parkinson's disease; every other change of use and of different purposes requires different tests and research from those of the present invention.

### Industrial Applicability

The present invention does not involve administrations of markers of any type to the patient but is based only on an immuno-enzymatic reaction (ELISA) performed on the (previously frozen) plasma of the patient (whereas obtaining the cerebrospinal fluid is very difficult because the drawing operation is dangerous and highly invasive for the patient) and uses a simple spectrophotometric final determination, which is the basis for the routine tests that are normally carried out in public and private clinical laboratories. Consequently, with the present method and with the related application kit it is possible both to diagnose the disease in an early stage thereof, and to start the related therapy early with a significant improvement of the quality of life of the patient.

## Claims

1. A method to identify the early stage of Parkinson's disease through the *in vitro* determination of the levels of the C-terminal peptide containing portion of the VGF protein present in plasma of a human subject, said method consisting in the following steps:
1) submitting the plasma of said subject to an ELISA assay which uses antibodies that specifically mark the VGF protein C-terminal peptide, said C-terminal peptide being represented by the amino acid sequence **-I EHVL LRRP;**
2) calculating the VGF protein levels by the spectrophotometric determination of the optical density of the samples obtained at the end of the ELISA immuno-enzymatic reaction of step 1), said spectrophotometric determination being calculated in absorbance at 495 nm.

2. The method of claim 1, in which in said step 1), during said performing of the ELISA assay, the plasma of the subject is reacted into a multiwell plate with a primary antibody, that specifically marks the human VGF C-terminal peptide - **1 EHVL LRRP** , at room temperature in duplicate, to give the corresponding protein-primary antibody conjugate.

3. The method of claims 1-2, in which said protein-primary antibody conjugate is then reacted with a biotinylated secondary antibody, that specifically marks said primary antibody, and with streptavidin peroxidase, to give the corresponding protein primary antibody-biotinylated secondary antibody conjugate and streptavidin peroxidase.

4. The method of claims 1-3, in which the optical density, i.e. the concentration of the samples containing said protein-primary antibody-biotinylated secondary antibody conjugate and streptavidin peroxidase, is measured by using a multilabelled plate reading spectrophotometer at room temperature and at 450 nm.

5. The process according to any one of claims 1-4, in which in the early stage Parkinson's patients, the levels of the VGF protein have a decrease of > 50% compared to healthy patients.

6. Use of the amino acid sequence -I EHVL LRRP in the identification of an early stage of Parkinson's disease, said identification being performed *in vitro* on plasma samples of a human subject by ELISA.

7. Use of the amino acid sequence according to claim 6 in which the levels of VGF protein of which said amino acid sequence is the C-terminal peptide, are in lower quantities in a subject suffering from an early stage of Parkinson's disease compared to a healthy subject.

8. Use of the amino acid sequence according to any of claims 6-7, wherein the levels of VGF protein, of which said amino acid sequence constitutes the C-terminal peptide, in patients with early stage Parkinson's are decreased by > 50% compared to healthy subjects.

## Patentansprüche

1. Verfahren zur Identifizierung des frühen Stadiums der Parkinson-Krankheit durch die *in vitro-Bestimmung* der Spiegel des c-terminalen Peptid-enthaltenden Teils des VGF-Proteins, das im Plasma eines menschlichen Subjekts vorhanden ist, wobei das Verfahren die folgenden Schritte umfasst:
1) Unterziehen des Plasmas dieses Subjekts einem ELISA-Test, der Antikörper verwendet, die spezifisch die c-terminalen Peptide des VGF-Proteins identifizieren, wobei solche c-terminalen Peptide durch die Aminosäuresequenz - I EHVL LRRP dargestellt werden;
2) Berechnen der VGF-Proteinspiegel durch die spektrophotometrische Bestimmung der optischen Dichte der am Ende der immunenzymatischen ELISA-Reaktion von Schritt 1 erhaltenen Proben, wobei diese spektrophotometrische Bestimmung mit einer Absorption von 495 nm berechnet wird.

2. Verfahren nach Anspruch 1, wobei in Schritt 1) während der Durchführung des ELISA-Tests das Plasma des Subjekts dazu gebracht wird, innerhalb eines Blutplättchens mit einer Vielzahl von Vertiefungen mit einem primären Antikörper zu reagieren, der spezifisch den menschlichen VGF c-terminalen Peptid 1 EHVL LRRP markiert, bei Raumtemperatur in zweifacher Ausfertigung, um das entsprechende primäre Antikörper-Protein-Konjugat zu bereitstellen.

3. Verfahren nach den Ansprüchen 1-2, wobei das primäre Antikörper-Protein-Konjugat wird dann mit einem biotinylierten sekundären Antikörper, der den primären Antikörper spezifisch markiert, und mit einer Streptavidinperoxidase umgesetzt wird, um das entsprechende sekundäre Antikörper-Konjugat des biotinylierten primären Protein-Antikörper und Streptavidinperoxidase zu bereitstellen.

4. Verfahren nach den Ansprüchen 1 bis 3, wobei die optische Dichte, d. h. die Konzentration der Proben, die das sekundäre Antikörper-Konjugat des biotinylierten primären Protein-Antikörper und Streptavidinperoxidaseprotein enthalten, unter Verwendung eines Objektträgers mit einer Vielzahl von Markierungen für eine spektrophotometrische Ablesung bei Raumtemperatur und bei 450 nm, gemessen wird.

5. Verfahren nach einem der Ansprüche 1 bis 4, wobei im frühen Stadium von Parkinson-Patienten die VGF-Proteinspiegel im Vergleich zu gesunden Patienten um mehr als 50% erniedrigt sind.

6. Verwendung der Aminosäuresequenz -I EVHL LRRP zur Identifizierung von Parkinson-Krankheit im frühen Stadium, wobei eine solche Identifizierung *in vitro* an Plasmaproben von einem Menschen durch ELISA durchgeführt wird.

7. Verwendung der Aminosäuresequenz nach Anspruch 6, wobei die Spiegel des VGF-Proteins, bei dem die Aminosäuresequenz den c-terminalen Peptiden entspricht, bei einem Patienten, der an einem frühen Stadium der Parkinson-Krankheit leidet, in geringeren Mengen vorliegen, im Vergleich zu einem gesunden Probanden.

8. Verwendung der Aminosäuresequenz nach einem der Ansprüche 6 bis 7, wobei die Spiegel des VGF-Proteins, bei dem die Aminosäuresequenz die c-terminalen Peptide darstellt, bei Patienten mit einem frühen Stadium der Parkinson-Krankheit mehr als 50% im Vergleich zu gesunden Probanden, verringert sind.

## Revendications

1. Procédé d'identification du stade précoce de la maladie de Parkinson par la détermination *in vitro* des niveaux de la partie contenant les peptides c-terminaux de la protéine VGF présente dans le plasma d'un sujet humain, lequel procédé comprenant les étapes suivantes:
1) soumettre le plasma de ce sujet à un test ELISA qui utilise des anticorps identifiant spécifiquement les peptides c-terminaux de la protéine VGF, ces peptides c-terminaux étant représentés par la séquence d'acides aminés -I EHVL LRRP;
2) calculer les niveaux de protéine VGF par une determination spectrophotométrique de la densité optique des échantillons obtenus à l'issue de la réaction immuno-enzymatique ELISA de l'étape 1), cette determination spectrophotométrique étant calculée avec une absorption de 495 nm.

2. Méthode selon la revendication 1, dans laquelle dans ladite étape 1) lors de l'exécution du test ELISA, le plasma du sujet est amené à réagir à l'intérieur d'une plaquette avec une multiplicité de puits avec un anticorps primaire, qui identifie spécifiquement les peptides c-terminaux humains VGF-1 EHVL LRRP, à température ambiante en double, pour donner le conjugué de l'anticorps proteique primaire correspondant.

3. Méthode selon les revendications 1-2, dans lequel ledit conjugué de l'anticorps proteique primaire est ensuite mis à réagir avec un anticorps secondaire biotinylé qui identifie spécifiquement ledit anticorps primaire, et avec une streptavidine peroxydase, pour donner le conjugué correspondant de l'anticoorps secondaire de l'anticorps protéique primaire biotinylé et de la peroxydase de streptavidine.

4. Procédé selon les revendications 1 à 3, dans lequel la densité optique, c'est-à-dire la concentration des échantillons contenant ledit conjugué de l'anticorps secondaire de l'anticorps protéique primaire biotinylé et de la peroxydase de streptavidine est mesurée à l'aide d'une plaquette avec une multiplicité de étiquettes pour une lecture spectrophotométrique à température ambiante et à 450 nm.

5. Procédé selon l'une quelconque des revendications 1 à 4, dans lequel, à l'étape initial des patients atteints de la maladie de Parkinson, les niveaux de protéine VGF sont diminués de plus de 50% par rapport aux patients sains.

6. Utilisation d'une séquence d'aminoacides -I EHVL LRRP dans l'identification de la maladie de Parkinson à une étape précoce, une telle identification étant effectuée *in vitro* sur des échantillons de plasma d'un sujet humain par ELISA.

7. Utilisation de la séquence d'aminocides selon la revendication 6, dans laquelle les niveaux de protéine VGF, dans laquelle ladite séquence d'aminoacides correspond aux peptides c-terminaux, sont en quantités moindres chez un sujet atteint d'un stade initial de la maladie de Parkinson, par rapport à un sujet sain.

8. Utilisation de la séquence d'aminoacides selon l'une quelconque des revendications 6 et 7, dans laquelle les niveaux de protéine VGF, dans laquelle ladite séquence d'aminoacides constitue les peptides c-terminaux, chez des patients présentant un stade précoce de la maladie de Parkinson, sont diminués par plus de 50% par rapport aux sujets sains.
